Europäisches Patentamt

European Patent Office

Office européen des brevets

(11) Numéro de publication: **0 354 849**
**A1**

# (12) DEMANDE DE BREVET EUROPEEN

(21) Numéro de dépôt: **89402258.1**

(22) Date de dépôt: **10.08.89**

(51) Int. Cl.⁵: **A 01 K 67/033**

(30) Priorité: **12.08.88 FR 8811015**

(43) Date de publication de la demande:
**14.02.90 Bulletin 90/07**

(84) Etats contractants désignés:
**AT BE CH DE ES FR GB GR IT LI LU NL SE**

(71) Demandeur: **CENTRE NATIONAL DE LA RECHERCHE
SCIENTIFIQUE (CNRS)
15, Quai Anatole France
F-75700 Paris Cedex 07 (FR)**

(72) Inventeur: **Gomot, Philippe
Peloussey "Aux Essarts"
F-25170 Recologne (FR)**

(74) Mandataire: **Ores, Irène et al
CABINET ORES 6, Avenue de Messine
F-75008 Paris (FR)**

(54) Procédé et dispositif pour l'élevage et la production industrielle des escargots.

(57) La présente invention concerne un procédé et un dispositif pour l'élevage et la production industrielle d'escargots.

Selon l'invention le procédé consiste à placer les animaux par petits groupes dans des cages spécifiques installées dans une enceinte conditionnée en température hygrométrie et éclairage selon des cycles et paramètres adaptés au comportement biologique des animaux, à appliquer une prophylaxie de haut niveau, et à nourrir les animaux à l'aide d'un aliment composé spécifique.

Le dispositif selon l'invention se présente sous la forme d'unmodule (1) muni d'une paroi latérale de ventilations (18') d'une porte frontale (3) équipée d'un opercule (4). Un nombre de modules assemblés les uns aux autres par les moyens (8) et (9) permettent de réaliser des ensembles de cages que l'on place horizontalement sur des supports adaptés à cet effet, un orifice (5) permet le passage d'une rampe de lavage, une gouttière (6) récupère les eaux de lavage.

L'invention trouve principalement son application dans le domaine de l'héliciculture.

Fig.1

Bundesdruckerei Berlin

**Description**

## Procédé et dispositif pour l'élevage et la production industrielle des escargots

La présente invention a pour objet un procédé et un dispositif pour l'élevage des escargots comprenant pour le procédé un nombre de moyens et de paramètres définis et pour le dispositif une cage se présentant sous la forme d'un cylindre juxtaposable à un même cylindre voisin pour constituer des ensembles de cages d'élevage.

Dans le domaine de la transformation et de la consommation de l'escargot si l'on sait que la France est encore aujourd'hui le premier pays mondial on sait également que la consommation de ce type de gastéropode tend à s'étendre de plus en plus largement è d'autres pays dans le monde.

La consommation d'escargots qui a commencé depuis l'antiquité a reposé très longtemps et repose encore à l'heure actuelle pour près de 100% sur un approvisionnement en animaux sauvages de ramassage et du fait de ce ramassage abusif les ressources françaises se sont totalement épuisées et l'on va chercher de plus en plus loin une matière première qui se raréfie de façon irréversible.

Devant ce problème, des tentatives de domestication sont apparues essentiellement en France afin de substituer un matière première d'élevage au produit naturel en régression qui cause de surcroît une importante fuite de devises en raison de son achat massif à l'étranger.

Les tentatives d'élevage d'escargot connues ont surtout concerné deux catégories d'animaux, l"escargot "petit gris" ou Hélix aspersa aspersa et "l'escargot de Bourgogne" ou Hélix pomatia car ces souches étaient naturellement présentes en France, cependant ces deux espèces souffrent l'une et l'autre d'un lourd handicap à l'égard du domaine économique; le "petit gris" n'intéresse qu'une part infime du marché international (2%) en raison de sa petite taille et "l'escargot de Bourgogne" très difficile à domestiquer ne peut être élevé actuellement de façon rentable.

Considérant les problèmes développés ci-avant certains éleveurs se sont alors tournés vers une autre variété de L'Hélix aspersa: l'Hélix aspersa maxima ou "gros gris d'Algérie" importé par eux directement d'Algérie où des populations existent à l'état naturel, l'intérêt essentiel de cette variété tient à sa taille deux fois supérieure à celle du "petit gris" ce qui correspond au calibre le plus demandé sur le marché (45%), là encore, en dépit de la répétition d'essais portant sur plusieurs saisons, ces quelques éleveurs rencontrèrent beaucoup d'échecs en raison du manque de maîtrise des phénomènes biologiques concernant les répercussions des paramètres d'environnement sur la reproduction et la croissance de cette variété et également en raison d'une technique d'élevage qui privilégiait l'utilisation de parcs extérieurs durant la belle saison en l'absence de matériel spécifique adapté à la biologie particulière de l'animal, dans ces conditions le plus grave problème rencontré par ces éleveurs est l'impossibilité d'obtenir des géniteurs d'élevage efficaces et fiables si bien que devant ce nouvel handicap ce type d'élevage reste pour une large part tributaire d'importations de souches sauvages d'Algérie à chaque nouvelle saison de reproduction.

On connaît également des dispositifs d'élevage hors sol de différents types dans lesquels les escargots sont placés en assez grands nombres dans des cages de volume suffisant mais ce type de conditionnement est nuisible pour la croissance et plus encore pour la reproduction en raison du regroupement fréquent des animaux en un seul point des cages par instinct naturellement grégaire.

La présente invention entend remédier à l'ensemble de ces inconvénients en proposant un procédé et un dispositif pour l'élevage des escargots permettant d'assurer une production ininterrompue et autonome d'animaux aux performances de reproduction et de croissance totalement nouvelles.

Issu de recherches scientifiques et d'expériences menées principalement sur l'influence des paramètres d'environnement sur le comportement de l'escargot, suivies d'applications expérimentales dépassant la vingtième génération d'animaux, le procédé selon l'invention permet d'obtenir les meilleurs résultats connus jusqu'à ce jour quant à la fiabilité dans la production de géniteurs excellents reproducteurs, à la réduction importante de la durée des cycles d'engraissements qui par ailleurs se succèdent sans interruption, et à la qualité du produit nouveau et totalement différent de la souche originelle entre autre par une texture de chair plus tendre et des qualités organoleptiques notoirement accrues.

A cet effet le procédé d'élevage selon l'invention est appliqué à la souche d'escargot "Helix aspersa maxima" ou gros gris d'Algérie, en raison de sa taille correspondant à la plus grande demande du marché, en tenant compte principalement de la physiologie de la reproduction et de la croissance de l'animal ainsi que des répercussions des paramètres d'environnement sur ces fonctions, sans toutefois que l'application dudit procédé se limite à cette seule souche de gastéropode.

Les résultats obtenus après expérimentation du procédé d'élevage selon l'invention ont par ailleurs permis de constater que l'on se trouvait en présence d'un nouvel individu aux comportements physiologique et biologique différents de la de la souche originelle, ce qui a entraîné la création d'une dénomination: le "BIOLIX" (marque déposée) afin de discerner ce nouvel individu parmi les autres gastéropodes des mêmes genre famille et variété.

De manière judicieuse le procédé d'élevage selon l'invention consiste à mettre en oeuvre un nombre de moyens et de paramètres comprenant principalement: un conditionnement en température et hygrométrie des locaux destinés à l'élevage, une répartition des animaux par petits groupes dans des dispositifs ou cages spécifiquement adaptés, une nourriture constituée d'un aliment composé spécifique facteur important de la croissance et des qualités organoleptiques de la production, une prophylaxie assurant entre autre le lavage périodique des cages d'élevage les débarrassant des excréments et des restes éventuels de nourriture souillée supprimant les risques de maladie et assurant par ailleurs un arrosage qui maintient l'activité des animaux favorisant leur prise de nourriture et empêchant leur collage contre sur les parois des cages, un éclairage artificiel irradiant lesdits animaux par photopériodes longues ou selon un mode séquentiel qui seront indiqués plus loin, et enfin, une organisation précise des phases de reproduction nurserie et engraissement permettant d'assurer la constance des objectifs de production

De façon judicieusement contrôlable les régulations physiologiques de la reproduction et de la croissance deviennent selon l'invention entièrement dépendantes des conditions d'environnement appliquées et constamment contrôlées, et le choix judicieux de ces conditions soit inhibitrices soit stimulatrices permet d'obtenir à la demande et à tout moment de l'année des cycles de reproduction et ou des cycles de croissance qui peuvent se succéder de manière ininterrompue, ce qui est totalement impossible à réaliser avec la variété originelle prélevée dans les sites d'origine. A cet effet, on notera par exemple qu'à la fin d'un cycle de croissance (appelé également cycle d'engraissement) de 49 jours seulement on obtient non seulement des escargots commercialisables, mais également des géniteurs qui peuvent être utilisés de suite en reproduction et dont le comportement ainsi que celui de leurs descendants est particulièrement bien adapté à l'application du procédé d'élevage selon l'invention.

De manière avantageuse, si une mise en repos temporaire des animaux était souhaitée pour une raison quelconque, par exemple pour un besoin passager de réduction de la production, l'arrêt du lavage conjointement à l'abaissement de la température et du taux d'hygrométrie facilement réalisables permettront d'apporter sans difficulté les résultats souhaités. Le redémarrage de l'activité des animaux se fera ensuite avec la même facilité en réajustant les paramètres modifiés à un niveau correspondant aux conditions de production.

On voit d'ores et déjà que le procédé d'élevage selon l'invention se prête particulièrement bien à l'implantation d'unité de production sous tout climat dans une parfaite autonomie de fonctionnement principalement en raison du fait qu'il permet la reproduction hors sol de géniteurs fiables ainsi qu'une production d'escargots en cycles ininterrompus.

Dans une variante d'application du procédé d'élevage selon l'invention, praticable dans les pays et/ou les régions dont les conditions climatiques le permettent, il est important de noter que ledit procédé pourra être étendu à une formule dite d'élevage mixte utilisant des parcs extérieurs durant la belle saison ce qui dans ce cas permettra une augmentation temporaire de la production et également la récolte éventuelle de géniteurs, lesdits parcs seront alors peuplés de jeunes animaux issus de la reproduction hors sol selon le procédé. Cependant il faut noter également que se les géniteurs fabriqués hors sol ne posent aucun problème de disponibilité et fiabilité, il n'en et pas de même pour les géniteurs fabriqués en parcs qui demandent une bonne sélection et une bonne programmation de leur période de repos et période d'activité.

Le procédé d'élevage selon l'invention sera mieux compris au cours de la description qui donnera plus loin, à titre d'exemple purement indicatif et non limitatif, une définition et une forme d'application des différents moyens et paramètres qui le composent.

Le dispositif d'élevage selon l'invention permet l'application du procédé décrit ci-avant et concourt de façon spécifique et rationnelle au succès de sa mise en oeuvre.

A cet effet le dispositif d'élevage selon l'invention se présente sous la forme générale d'un module de base ou unité de cage juxtaposable horizontalement à un même module voisin lequel deuxième module est juxtaposable à un troisième identique aux deux précédents et ainsi de suite pour former des groupes par exemple de 10, 15 ou 20 cages voire plus dont les deux extrémités seront ensuite équipées d'un axe permettant de reposer les groupes de cases ainsi réalisés dans des demi paliers placés eux mêmes sur des consoles murales ou centrales à plusieurs niveaux afin de permettre de conditionner des ensembles de cages et des installations d'élevages de toutes importance et dimensions.

Selon l'invention le dispositif est constitué d'un module réalisé par injection de matière synthétique transparente et de qualité alimentaire, d'un volume habitable par exemple d'environ 18 litres et d'une surface totale par exemple de a,40 mètre carré muni d'une paroi latérale à l'une des extrémités, d'une gouttière de récupération des eaux de lavage formant saillie longitudinale sur le diamètre extérieur dudit module, d'une porte d'accès équipée d'un opercule destiné à l'introduction des aliments. La paroi latérale comporte un orifice coaxial apte à recevoir le passage d'une rampe de lavage.

De manière avantageuse l'extrémité du module munie d'un fond comporte une partie rétrécie permettant son emboîtage et son collage dans l'autre extrémité d'un même module voisin. Le dernier module d'un ensemble de modules ainsi juxtaposés est fermé par un fond réalisé à cet effet sous une forme identique à la partie rétrécie du dispositif afin de former la dernière cage.

Dans une forme préférée de réalisation de l'invention les ensembles constitués de cylindres ou cages assemblés comme décrit précédemment se présentent sous la forme d'un tube compartimenté formant une série de cages dont les portes sont alignées sur le même axe et la gouttière assemblée de façon continue et étanche sur la longueur totale du tube, lesdits tubes sont traversés de façon sensiblement coaxiale par une rampe de lavage munie d'une pluralité de becs permettant d'assurer le nettoyage de chaque cage. Les ensembles de cages ainsi réalisés sont placés et supportés horizontalement par les axes situés à leurs extrémités dans des demi-paliers solidaires des supports afin de permettre l'orientation des cages selon les besoins de nettoyage d'entretien ou de récolte des animaux.

Dans une variante plus économique de réalisation de l'invention les tubes ou ensembles de cages décrits au paragraphe précédent ne comportent ni axe ni palier support et sont supportés par des sangles fixées par exemple contre les parois des locaux ou contre des poteaux dans le cas d'implantation centrale.

Enfin, le cylindrique, module de base ou unité de cage peut être réalisé sous une forme polygonale et/ou troncônique sans sortir du cadre ni du principe de l'invention.

Le procédé et le dispositif selon l'invention seront mieux compris au cours de la description donnée ci-après à titre d'exemple purement indicatif et non limitatif qui permettra d'en dégager les avantages et les caractéristiques secondaires.

Pour le procédé il sera fait état des différents moyens et paramètres qui en font la spécificité.

Pour le dispositif il sera fait référence aux dessins annexés dans lesquels:

    - La figure 1 illustre par deux vues en coupe le dispositif selon un mode préféré de réalisation de l'invention.

    - La figure 2 montre par une vue en coupe un ensemble de dispositifs selon la figure 1, assemblés entre eux.

    - La figure 3 montre par une vue de face et une vue en coupe un axe de repos d'un ensemble de dispositifs selon la figure 2 .

    - La figure 4 montre par une vue de côté et une vue partielle de face un ensemble de dispositifs en installation murale.

    - La figure 5 montre par une vue en côté un ensemble de dispositifs en installation centrale.

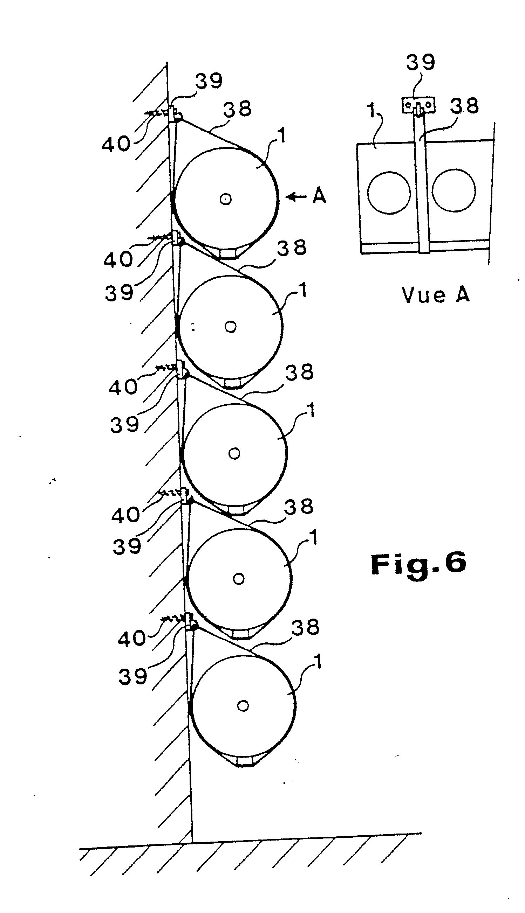

    - La figure 6 montre par une vue de côté un ensemble de dispositifs en installation murale selon une variante de réalisation de l'invention.

On commencera tout d'abord par la description du procédé.

Le procédé d'élevage selon l'invention comprend un nombre de moyens et de paramètres étudiés et testés scientifiquement dont une forme préférée de l'application est donnée dans la description ci-après à titre d'exemple purement indicatif et non limitatif.

Après la phase ponte éclosion et nursurie que l'on verra plus loin à la fin du premier cycle d'élevage, les escargots issus de nurserie où ils ont séjourné environ 28 à 42 jours après leur éclosion sont répartis pour la phase dite d'engraissement à raison de 40 à 50 individus dans des cages d'environ 18 litres de volume et d'une surface totale d'environ 0,40 mètre carré conçues pour permettre la pénétration de la lumière à leur intérieur et groupées dans une enceinte contrôlée et conditionnée artificiellement pour apporter les différents paramètres dits d'environnement comprenant pour la phase d'engraissement et de croissance: une température de 24 à 26 degrés celsius, une hygrométrie de 95 à 100 %, une émission artificielle de lumière type lumière du jour la plus proche possible du spectre naturel distribuée selon des photopériodes longue de 16 à 18 heures par 24 heures ou selon un mode séquentiel comprenant une phase longue de 7 à 8 heures suivie d'une phase courte de 2 heures intercalées par des périodes obscures d'environ 4 heures, une prophylaxie de très haut niveau assurant entre autre une élimination périodique des excréments à l'aide d'un moyen automatique de lavage des cages et un arrosage des animaux assurant également leur maintien en activité favorisant ainsi leur prise de nourriture, une organisation précise de l'élevage permettant d'assurer la constance des objectifs de production se situant au niveau de la répartition et de la destination des cages d'élevage à raison de 12 à 15 cages de reproduction et 10 à 13 cages de nurserie pour 100 cages d'engraissement, et une nourriture constituée d'un aliment composé spécifique.

A la fin du cycle d'engraissement de 49 jours seulement, lorsque les escargots sont d'une taille et d'un poids qui en font un produit commercialisable, on procède alors à la récolte et dans cette même récolte à la sélection et au prélèvement des géniteurs qui se fait parmi les animaux bordés (l'animal est dit bordé lorsque le bord libre de sa coquille forme un bourrelet dur attestant que l'escargot a terminé sa croissance en taille alors qu'une partie de l'élevage peut n'avoir atteint qu'un niveau de croissance l'amenant seulement à son poids de commercialisation) ces géniteurs seront ensuite placés directement en phase de reproduction sans avoir besoin de passer par une période d'hibernation, il est cependant important de noter ici que ces géniteurs se prêtent également à l'hibernation par exemple en cas de besoin lié aux nécessités d'organisation de l'élevage il est possible de recourir à cette méthode et dans ce cas la géniteur élevé selon le procédé donnera de bons résultats après seulement deux mois d'hibernation à une température de 7 degrés celsius.

Après leur prélèvement les géniteurs sont placés en conditions de reproduction à une température comprise entre 15 et 20 degrés celsius, dans des cages identiques aux cages d'engraissement mais communiquant cette fois entre elles par groupe de trois par des orifices ménagés à cet effet dans les cloisons séparatrices, 30 à 40 géniteurs sont répartis dans les cages d'extrémité d'un groupe de trois cages et un bac de ponte est placé dans la cage centrale de laquelle on condamne le système de lavage. On notera ici en ce qui concerne l'éclairement des animaux, que la lumière rouge favorise la ponte et la lumière jaune favorise les accouple ments et que tant pour la reproduction que pour l'engraissement la puissance d'éclairage pourra se situer dans une très large gamme comprise entre 150 et 2000 lux. Environ 8 jours après leur éclosion les jeunes animaux sont placés par groupes de 280 à 320 individus par cage identiques aux cages d'engraissement mais dites de nurserie pour la circonstance où ils séjourneront pendant 28 à 42 jours avant d'être repartis à raison de 40 à 50 individus par cage pour un nouveau cycle d'engraissement.

Dans une variante d'application du procédé selon l'invention et si les conditions climatiques du lieu ou se situe l'élevage s'y prêtent, on pourra étendre la mise en oeuvre dudit procédé à une formule dite mixte en utilisant des parcs extérieurs durant la belle saison ce qui permettra une augmentation temporaire de la production pouvant aller jusqu'à deux cycles de production en parc par saison avec également des récoltes possibles de géniteurs. Dans ce cas les animaux sont placés dans les parcs à raison d'environ 350 individus au mètre carré à partir d'escargot issus de 4 semaines de la nurserie de l'élevage hors sol ou à raison de 400 individus par mètre carré à partir d'animaux âgés de 8 jours après leur éclosion. les géniteurs récoltés en parc étant issus de la couche obtenue hors sol selon la procédé, pourront être placés dans les conditions requises et utilisés de suite en reproduction ou être mis en repos à température ambiante, période dite d'estivation dont la durée ne devra pas dépasser 3 mois, ou encore être placés en hibernation en chambre froide à une température d'environ 7 degrés celsius dont la durée pourra atteindre 6 à 8 mois. Toutefois il faut préciser que pour obtenir de bons résultats en reproduction après une période d'hibernation avec des géniteurs récoltés en parc la période d'hibernation devra être différente selon que les géniteurs se sont bordés dans une période courte, moyenne, ou longue. Un géniteur bordé dans un délais court donnera de bons résultats après une hibernation de trois mois seulement alors qu'un géniteur bordé dans un délai moyen ou long ne donnera de bons résultats en reproduction qu'après une période d'hibernation au moins égale à 6 mois, l'utilisation des géniteurs récoltés en parc demandera donc une bonne programmation de leurs périodes de repos et d'activité afin de ne pas perturber les besoins de la production.

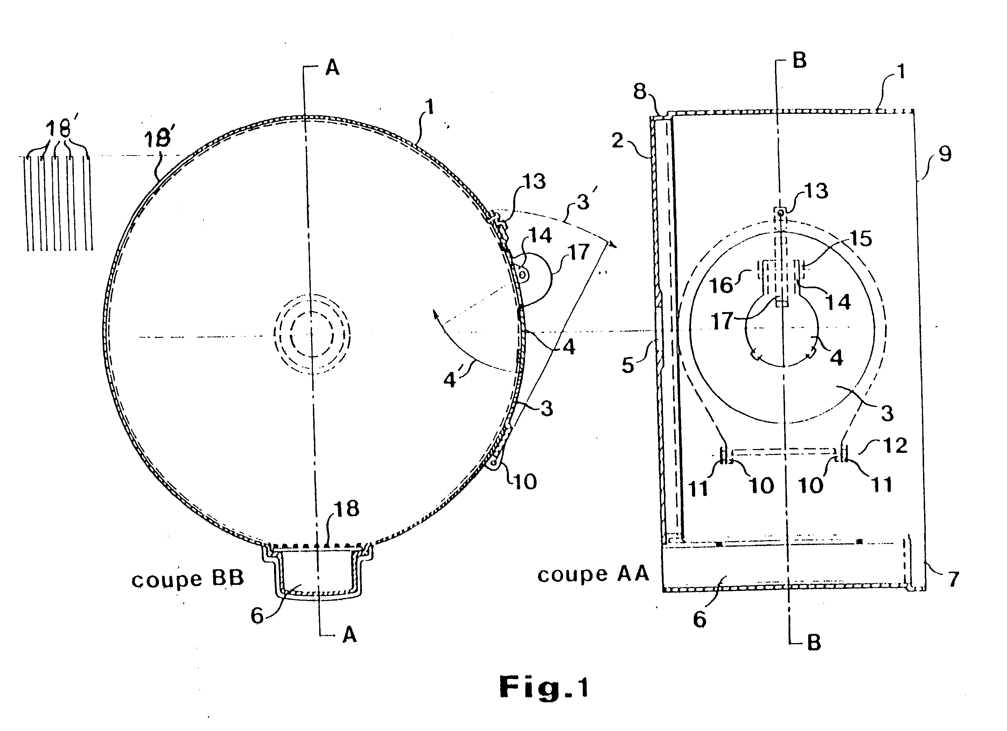

Pour la description du dispositif d'élevage selon l'invention on se reportera tout d'abord à la figure 1 sur laquelle on voit en coupe AA et BB le cylindre (1) constituant une unité de cage d'élevage juxtaposable à un même cylindre (1) voisin et ainsi de suite pour réaliser des ensembles de cages d'élevage destinées aux logement des animaux, comme on le voit sur cette figure ledit cylindre (1) comporte, une gouttière (6) de récupération des eaux de lavage, une grille (18)empêchant les animaux de tomber dans ladite gouttière (6), une paroi latérale (2), une porte 3) ouvrant vers l'extérieur salon (3') équipée d'un moyen de fermeture (13)et d'un percule (4) ouvrant vers l'intérieur selon (4') équipée lui même d'unressort (1è) apte à assurer et maintenir sa fermeture, la porte (3) comporte de façon solidaire au moins deux chapes (10) reliées par au moins un axe (12) à au moins deux xhapes (11) elles mêmes solidaires du module (1) de manière à assurer son articulation d'ouverture, et l'opercule (4) comporte de façon solidaire au moins un moyen (14) relié par au moins un axe (16) à au moins deux chapes (15) solidaires de la porte (3)de manière à assurer son articulation d'ouverture.

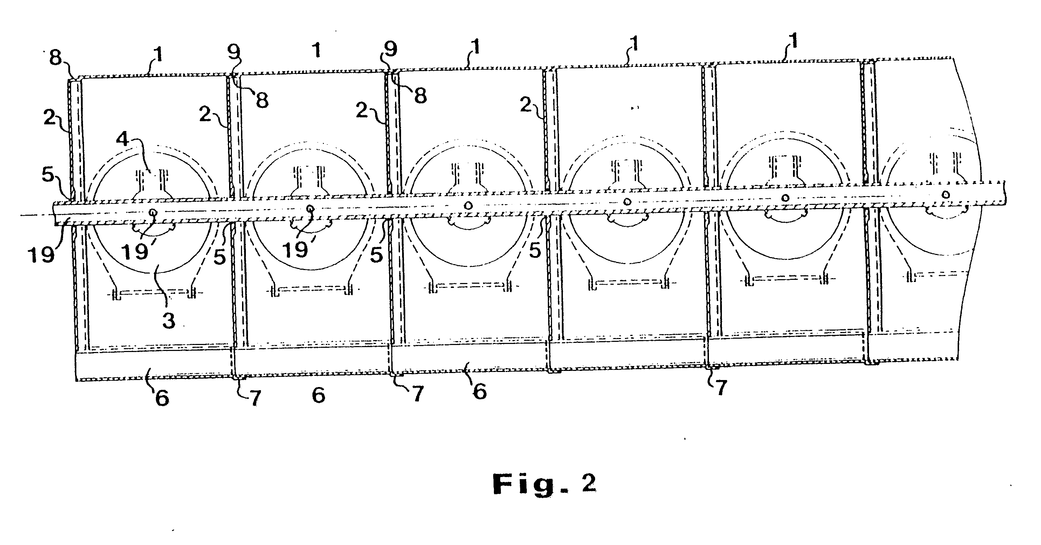

Sur cette même figure (1) on voit enfin que le cylindre (1) comporte une partie rétrécie (8) et la gouttière (6) un moyen (7) permettant l'assemblage d'une pluralité de cylindre (1) entre eux par emboitage et collage de l'extrémité (8) de chaque cylindre (1) dans l'extrémité (9) de chaque cylindre (1) voisin, l'extrémité (9) du dernier cylindre d'un ensemble ainsi réalisé est obturée par un fond non représenté mais de dimensions identiques à celles de la paroi latérale (2) et ne comportant qu'un rebord correspondant à la longueur de la partie (8) afin de permettre son emboitage et son collage. La paroi latérale (2) est muni d'un orifice coaxial (5) permettant le passage d'une rampe de lavage comme on le verra et comprendra mieux sur la figure 2, et éventuellement d'un orifice permettant la circulation des escargots entre 2 cages. Des orifices (18') de forme non limitative et d'une surface appropriée sont destinés à la ventilation.

Sur la figure 2 qui représente un ensemble de cylindres (1) selon la figure 1 assemblée entre eux, on voit les assemblages réalisés par emboîtage des parties (8) et (9) des cylindres (1) voisins assurant dans le même temps l'emboîtage et l'étanchéité des gouttières (6) par le moyen (7) constituant ainsi un collecteur général de récupération des eaux le lavage de chaque cage. On remarque également le rampe (19) de distribution d'eau de lavage traversant l'ensemble des cages ou cylindres (1) par les orifices (5) ménagés à cet effet dans les parois latérales et les orifices (19') destinés à recevoir des becs ou gicleurs.

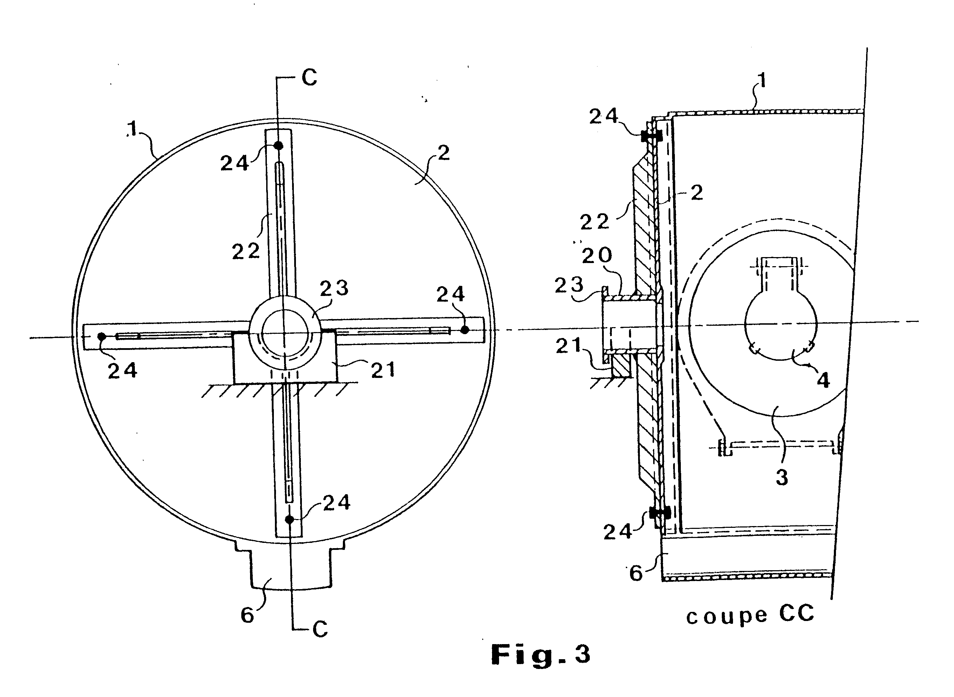

Sur la figure 3 qui représente une forme préférée de réalisation des supports d'ensembles de cylindres ou cages assemblés selon la figure 2, on voit l'axe tubulaire (20) solidaire de quatre bras (22) formant croisillon fixés chacun par leur extrémité sur la paroi latérale du cylindre (1) à l'aide de moyens (24), le demi-palier (21) servant de support à l'axe (20) et la bague (23) solidaire de l'axe (20) assurant son maintien en place dans le demi-palier (21). L'axe (20 est creux pour permettre le passage de la rampe de lavage par son centre.

On voit d'ores et déjà qu'un ensemble de cylindres ou cages d'élevage assemblés selon la figure 2 et supporté horizontalement par ses deux extrémités à l'aide d'axes (20) et de demi-paliers (21) tels que représentés sur la figure 3 apporte une solution avantageuse au dispositif selon l'invention, car cet ensemble de moyens permet l'orientation des ouvertures des cages sur 360 degrés d'angle ce qui est appréciable par exemple pour faciliter les travaux d'entretien, de nettoyage et de récolte des animaux, et également pour déplacer une partie de l'élevage ou remplacer aisément un groupe de cages.

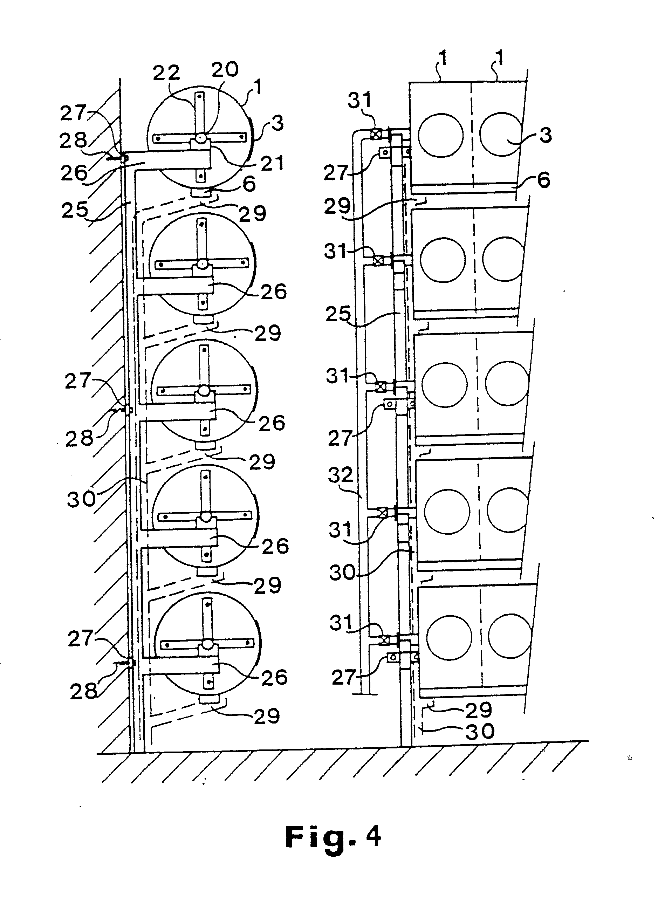

Sur la figure 4 qui illustre une forme préférée d'installation murale de dispositifs selon l'invention, on voit en vue de côté et en vue partielle de face un nombre non limitatif d'emsembles de cages ou cylindres (1) assemblés selon la figure (2) superposés sur un support mural constitué d'un montant vertical (25) muni de console (26) supportant chacune un demi-palier (21) dans lequel repose l'axe (20) équipant l'une des extrémités des ensembles de cages. Le montant (25) est muni de moyens (27) permettant d'assurer sa fixation contre une paroi à l'aide des moyens (28). Un même support (25) équipé de mêmes consoles (26) supporte par les mêmes moyens (20 21) et (22) les autres extrémités des ensembles de cages.

Sur cette même figure 4 on voit également sur la vue de côté les canalisations d'écoulement des eaux de lavage, dessinées ici en traits discontinus pour faciliter la lecture du dessin, constituées d'un collecteur (30) sur lequel se raccordent les goulottes (29) de récupération des écoulements des gouttières (6) de chaque niveau de cages, et sur la vue partielle de face on voit la colonne (32) de distribution d'eau de lavage à chaque niveau de cages et les vannes (31) qui peuvent être indifféremment manuelles, ou motorisées dans le cas d'automatisation du lavage.

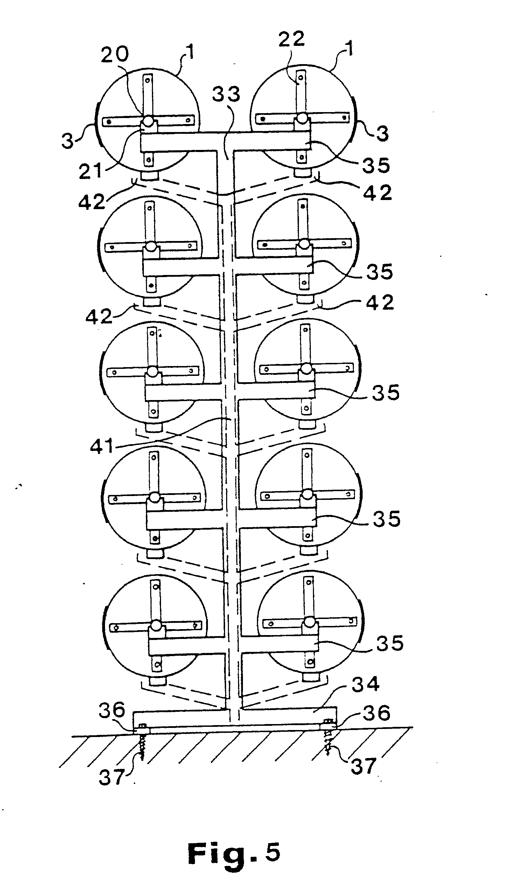

Sur la figure 5 qui représente une installation centrale d'ensembles de cages ou cylindres (1) assemblés entre eux et équipés des mêmes moyens (20) (21) et (22) que dans le cas d'installation murale selon la figure 4, on voit l'un des deux supports d'extrémité constitué d'un poteau central (33) muni de consoles (35) sur lesquelles reposent les demi-paliers (21) et équipé d'une traverse basse (34) de repos au sol munie elle même de moyens de fixation (36) et (37), et en traits discontinus pour faciliter la lecture du dessin le collecteur (41) de récupération des eaux de lavage sur lequel se raccordent les goulottes (42) de récupération des écoulements des gouttières (6) de chaque niveau de cages.

Sur la figure (6) qui représente une variante économique d'installation d'ensembles de dispositifs selon l'invention, on voit les ensembles de cages ou cylindres (1) superposés et supportés chacun par une sangle (38) dont les extrémités sont accrochées à un support mural (39) en forme de crochet équipé de moyens de fixation (40) dans ce cas on remarquera que l'on a plus besoin des moyens (20) (21) et (22) de la description se rapportant aux figures 4 et 5.

Enfin comme on l'a déjà dit dans la première partie de la description, le cylindre (1) formant élément de base ou unité de cage pourra également être réalisé sous une forme polygonale et/ou tronconique sans sortir du cadre ni du principe de la présente invention.

L'invention est avantageuse par le fait qu'elle apporte une solution efficace et fiable dans la production industrielle d'escargots et aussi par le fait qu'elle apporte un moyen rationnel et d'une grande souplesse d'utilisation permettant de réaliser aisément des installations industrielles d'élevage et de production d'escargots à une époque où ce prooduit est importé dans notre pays à près de 100% de notre consommation, et où de plus les ressources naturelles s'épuisent de façon irréversible.

L'invention trouve principalement son application dans l'élevage et la production industrielle et artisanale d'escargots.

Elle n'est pas limitée à la description qui vient d'en être donnée mais couvre au contraire toutes les variantes qui pourraient lui être apportées sans sortir de son cadre ni de son objet

## Revendications

1°) Procédé pour l'élevage et la production industrielle des escargots dans des enceintes artificiellement conditionnées en température, hygrométrie et éclairage, lesquelles variables sont programmées et contrôlées selon les exigences comportementales et physiologiques des animaux, et dans lequel on applique à l'élevage des mesures prophylactiques comprenant entre autre un lavage périodique des cages, lequel procédé est caractérisé en ce que les escargots sont répartis dans des cages de 16 à 20 litres de volume et de 0,35 à 0,40 mètres carrés de surface totale installées dans les enceintes conditionnées, et en ce qu'il comprend trois phases

a) une phase d'engraissement et de croissance pour laquelle les animaux sont répartis à raison de 40 à 50 individus par cage et nourris à l'aide d'un aliment composé spécifique, et à la fin de laquelle on procède à la récolte des animaux

b) une phase de reproduction, au cours de laquelle on place des géniteurs, sélectionnés parmi les animaux récoltés en fin de phase de croissance, dans des cages communiquant entre elles par groupe de trois, dont l'une est équipée d'un pondoir, à raison de 30 à 40 animaux par cage, et pendant laquelle on procède à la récolte des pontes.

c) une phase de nurserie, d'une durée de 28 à 42 jours, dans laquelle les animaux issus de la nouvelle ponte sont répartis à raison de 280 à 320 individus par cage, et à la fin de laquelle les animaux sont prélevés et placés en phase d'engraissement et de croissance.

2°) Procédé d'élevage selon la revendication 1, caractérisé en ce que, à l'issue de la phase de nurserie, les animaux sont placés, pour la phase d'engraissement, en parcs extérieurs.

3°) Procédé d'élevage selon la revendication 1, caractérisé en ce que, à l'issue de la phase d'engraissement, les animaux sélectionnés comme géniteurs sont, préalablement à la phase de reproduction, mis en repos à température ambiante.

4°) Procédé d'élevage selon la revendication 1, caractérisé en ce que, à l'issue de la phase d'engraissement, les animaux sélectionnés comme géniteurs sont, préalablement à la phase de reproduction, placés en hibernation en chambre froide.

5°) Procédé d'élevage selon la Revendication 1, caractérisé en ce que, à l'issue de la phase d'engraissement, les animaux sont placés immédiatement en conditions de reproduction.

6°) Procédé d'élevage selon la revendication 1, caractérisé en ce que les escargots sont soumis à un éclairement par photopériodes de 16 à 18 heures.

7°) Procédé d'élevage selon la revendication 1, caractérisé en ce que les cages d'élevage contenant les animaux en phase d'engraissement et de croissance ainsi que les cages d'élevage contenant les animaux en phase de nurserie sont placées dans une enceinte conditionnée artificiellement à une température ambiante de 23 à 26 degrés Celsius sous une hygrométrie de 95 à 100%.

8°) Procédé d'élevage selon la revendication 1, caractérisé en ce que les cages contenant les géniteurs en phase de reproduction sont placées dans une enceinte conditionnée à une température située entre 15 et 20 degrés celsius.

9°) Procédé selon la revendication 1, caractérisé en ce qu'on utilise, pour 100 cages d'engraissement, 12 à 15 cages de reproduction et 10 à 15 cages de nurserie.

10°) Dispositif d'élevage pour la mise en oeuvre du procédé selon l'une quelconque des revendications 1 à 9, formé de cages modulaires assemblables par juxtaposition horizontale, caractérisé en ce que chaque module, de forme tubulaire à section circulaire ou polygonale, ou de forme tronconique, comporte :

a) à l'une de ses extrémités, une partie rétrécie permettant son assemblage par emboitement avec le module voisin, ladite extrémité étant pourvue d'une paroi assurant la séparation transversale entre deux cages, ladite paroi étant en outre pourvue d'au moins un orifice permettant le passage d'un axe et/ou d'une rampe de lavage et éventuellement d'un orifice permettant la circulation des animaux d'un module à un autre.

b) une gouttière de récupération des eaux de lavage, pouvant être assemblée avec la gouttière du module voisin, de façon à former une gouttière longitudinale continue, commune à un ensemble de cages modulaires

c) une porte frontale, munie d'un opercule.

11°) Dispositif selon la revendication 10, caractérisé en ce que, à l'extrémité dépourvue de paroi latérale du dernier module d'un ensemble de cages, on adapte une paroi de forme identique à celle des parois latérales, éventuellement percée d'un orifice permettant le passage de l'axe.

12°) Dispositif selon la revendication 10 ou 11, caractérisé en ce qu'un ensemble de cages modulaires assemblées est traversé par un axe horizontal central passant par l'orifice ménagé dans la paroi latérale de chaque module, et dont les extrémités reposent sur un demi-palier adapté à un support, ledit assemblage étant réalisé de façon à permettre une rotation de 360° des cages sur l'axe central.

13°) Dispositif selon la revendication 10 ou 11, caractérisé en ce que l'ensemble résultant de la juxtaposition des cages modulaires est supporté par des sangles.

14°) Dispositif selon l'une quelconque des revendications 10 à 13, caractérisé en ce que plusieurs ensembles de cages assemblées sont fixés sur plusieurs niveaux horizontaux.

15°) Dispositif selon l'une quelconque des revendications 10 à 13, caractérisé en ce que les modules ont une forme cylindrique.

coupe BB

coupe AA

**Fig.1**

EP 0 354 849 A1

Fig. 2

coupe CC

**Fig. 3**

EP 0 354 849 A1

**Fig. 4**

**Fig. 5**

Vue A

**Fig.6**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | BE-A-893734 (BODART)<br>* le document en entier *<br>--- | 1, 10 | A01K67/033 |
| A | FR-A-2196747 (INSTITUT NATIONAL DE LA RECHERCHE AGRONOMIQUE)<br>--- | | |
| A | FR-A-2426408 (AUER)<br>----- | | |

**DOMAINES TECHNIQUES RECHERCHES (Int. Cl.5 )**

A01K

Le présent rapport a été établi pour toutes les revendications

| Lieu de la recherche | Date d'achèvement de la recherche | Examinateur |
|---|---|---|
| LA HAYE | 24 OCTOBRE 1989 | VON ARX V.U. |